# EUROPEAN PATENT APPLICATION

(11) **EP 3 925 554 A1**
(43) Date of publication of application: **22.12.2021**
(21) Application number: 21180227.7
(22) Date of filing: 18.06.2021
(51) Int. Cl.: A61B 17/32

(54) **SURGICAL SYSTEM COMPRISING A COOLING DEVICE**

(30) Priority: 18.06.2020 US 202063040630 P; 20.05.2021 US 202117325774
(71) Applicant: Covidien LP, Mansfield, MA 02048 (US)
(72) Inventor: COWLEY, Matthew, S, Boulder, CO Colorado 80301 (US); LYONS, Michael, B, Boulder, CO Colorado 80301 (US); VAN TOL, David, J, Boulder, CO Colorado 80301 (US)
(74) Representative: Maschio, Antonio

(57) **Abstract**

A surgical system includes a surgical instrument and a cooling module operably coupled to the surgical instrument. The surgical instrument includes a housing and an elongated portion extending from the housing and supporting an end effector. The cooling module includes a fluid reservoir retaining a cooling fluid, a thermoelectric cooler, and a pump assembly. The thermoelectric cooler has a first portion thermally coupled to the fluid reservoir and a second portion including a plurality of fins dissipating heat therefrom. The pump assembly is configured to supply the cooling fluid through at least a portion of the surgical instrument and back to the fluid reservoir, thereby defining a flow path to cool the end effector of the surgical instrument.

## Description

### FIELD

The present disclosure relates to surgical instruments and, more particularly, to devices for cooling a surgical instrument and a method of use thereof.

### BACKGROUND

Various types of energy (e.g., electrical, ultrasonic, microwave, cryogenic, thermal, laser, etc.) may be applied to tissue to achieve a desired result. Ultrasonic energy, for example, may be delivered to tissue to treat, e.g., coagulate and/or cut, tissue.

Ultrasonic surgical instruments typically include a waveguide having a transducer coupled thereto at a proximal end of the waveguide and an end effector disposed at a distal end of the waveguide. The waveguide transmits ultrasonic energy produced by the transducer to the end effector for treating tissue at the end effector. The end effector may include a blade, hook, ball, shears, etc., and/or other features such as one or more jaws for grasping or manipulating tissue. During use, the waveguide and/or end effector of an ultrasonic surgical instrument can reach temperatures greater than 200° C.

### SUMMARY

As used herein, the term "distal" refers to the portion that is being described which is farther from a user, while the term "proximal" refers to the portion that is being described which is closer to a user. Further, to the extent consistent, any of the aspects described herein may be used in conjunction with any or all of the other aspects described herein.

In accordance with the disclosure, a surgical system includes a surgical instrument and a cooling module operably coupled to the surgical instrument. The surgical instrument includes a housing and an elongated portion extending from the housing and supporting an end effector. The cooling module includes a fluid reservoir retaining a cooling fluid, a thermoelectric cooler, and a pump assembly. The thermoelectric cooler has a first portion thermally coupled to the fluid reservoir and a second portion including a plurality of fins dissipating heat therefrom. The pump assembly is configured to supply the cooling fluid through at least a portion of the surgical instrument and back to the fluid reservoir, thereby defining a flow path to cool the end effector of the surgical instrument.

In an aspect, the cooling module may be external of the surgical instrument.

In another aspect, the fluid reservoir may include dividers to guide flow of the cooling fluid.

In yet another aspect, the cooling module may be disposed within the housing of the surgical instrument.

In an aspect, the surgical instrument may include an ultrasonic waveguide having a blade at a distal end thereof.

In another aspect, the flow path may extend at least partially through the blade.

In yet another aspect, the surgical instrument may further include an ultrasonic transducer coupled to the ultrasonic waveguide to energize the blade for treating tissue.

In still yet another aspect, the surgical system may further include a generator configured to supply energy to the surgical instrument.

In still yet another aspect, the generator may be disposed on the surgical instrument.

In an aspect, the generator may be spaced-apart from the surgical instrument.

In another aspect, the surgical system may further include a controller configured to determine a temperature of the surgical instrument.

In yet another aspect, the controller may be configured to control the pump assembly.

In accordance with another aspect of the disclosure, a surgical system includes a surgical instrument and a cooling module. The surgical instrument includes a housing, an end effector operably coupled to the housing, and first and second conduits thermally coupled to the end effector. The cooling module is disposed within the housing. The cooling module includes a fluid line, a cooling fluid, a thermoelectric cooler, and a pump assembly. The fluid line retains a cooling fluid therein and interconnects the first and second conduits. The fluid line is in fluid communication with the first and second conduits thereby defining a closed fluid path. The cooling fluid is disposed in the closed fluid path. The thermoelectric cooler has a first portion thermally coupled to the fluid line and a second portion including a plurality of fins configured to dissipate heat therefrom. The pump assembly is configured to circulate the cooling fluid through the closed fluid path to cool the end effector of the surgical instrument.

### BRIEF DESCRIPTION OF DRAWINGS

Various aspects and features of the present disclosure are described hereinbelow with reference to the drawings wherein like numerals designate identical or corresponding elements in each of the several views:
FIG. 1 is a perspective view of a surgical system in accordance with the present disclosure including an ultrasonic surgical instrument, a cooling module, and a cooling system incorporated therein;
FIG. 2 is an enlarged, perspective view of a distal end of the ultrasonic surgical instrument of FIG. 1;
FIG. 3 is a schematic view of the surgical system of FIG. 1 depicting internal operating components of the cooling system thereof;
FIG. 4 is a schematic view of the cooling assembly of the surgical system of FIG. 1;
FIG. 5 is a schematic view of another cooling assembly for use with the surgical system of FIG. 1;
FIG. 6 is a schematic view of yet another cooling assembly for use with the surgical system of FIG. 1;
FIG. 7 is an exploded, perspective view of another surgical system provided in accordance with the present disclosure including another ultrasonic surgical instrument and an on-board cooling module having a cooling system incorporated therein; and
FIG. 8 is a flow diagram depicting a method of cooling an ultrasonic surgical instrument in accordance with the present disclosure.

### DETAILED DESCRIPTION

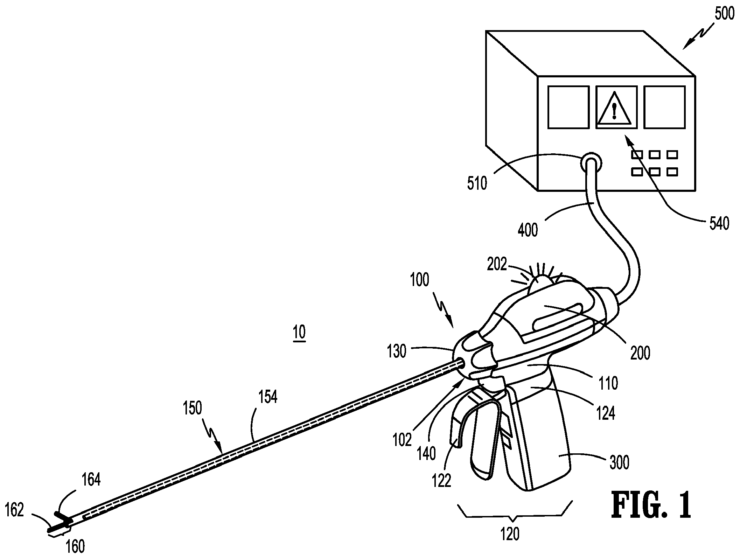

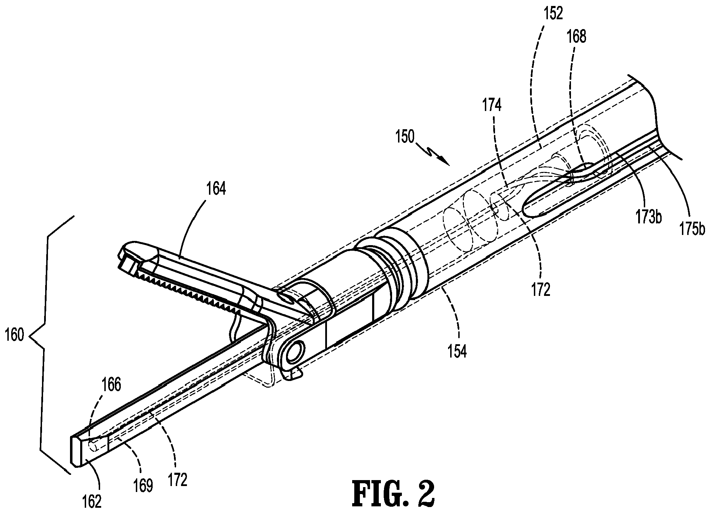

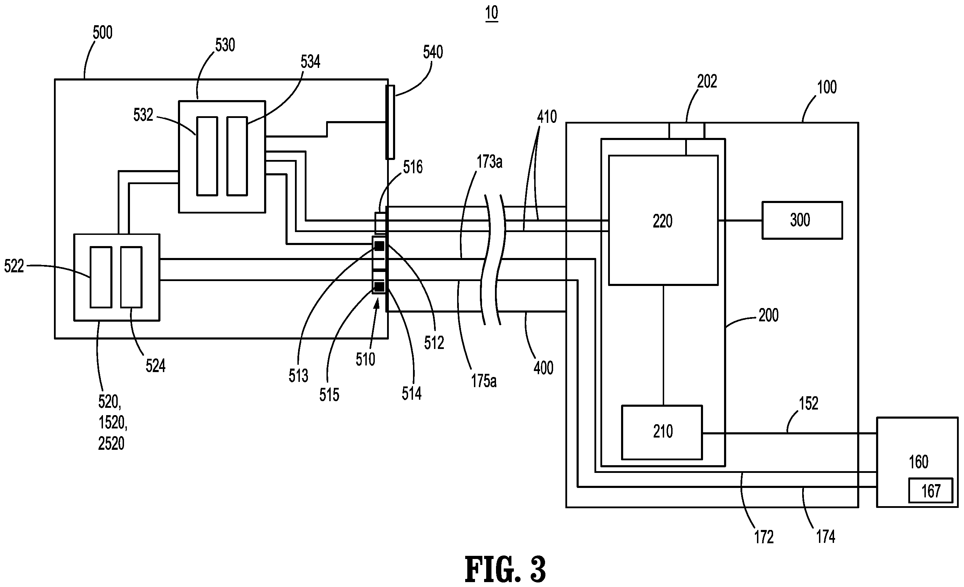

With reference to FIGS. 1 and 2, a surgical system in accordance with the present disclosure is shown generally as 10. The surgical system 10 generally includes an ultrasonic surgical instrument 100 and a cooling module 500 that, together, incorporate a cooling system for cooling a blade 162 of an end effector assembly 160 of the ultrasonic surgical instrument 100. The ultrasonic surgical instrument 100 generally includes a disposable 102, a transducer and generator assembly ("TAG") 200 including a transducer 210 and a generator 220 (FIG. 3), a battery 300, and a cable 400. Alternatively, ultrasonic surgical instrument 100 may be configured to connect to a remote generator and power source, thus eliminating the need for on-board generator 220 (FIG. 3) and battery 300. The disposable 102 includes a handle assembly 120, an elongated body portion 150, and the end effector assembly 160 supported on the elongated body portion 150. The TAG 200 is configured to convert electrical energy provided by the battery 300 into mechanical energy that is transmitted along a waveguide 152 to the blade 162. The TAG 200 and the battery 300 may be releasably engageable with a housing 110 of a handle assembly 120 and, when engaged therewith, the TAG 200 and the battery 300 are disposed in electrical communication with one another such that power and/or control signals can be relayed between the TAG 200 and the battery 300 for operating the ultrasonic surgical instrument 100. The TAG 200 may further include an indicator 202 disposed thereon, as will be described below.

The elongated body portion 150 of the ultrasonic surgical instrument 100 includes the waveguide 152 (FIG. 2) which extends from the housing 110 to the end effector assembly 160, an outer tube 154, and an inner tube (not shown). The distal end of waveguide 152 extends distally from the outer tube 154 and defines the blade 162 of the end effector assembly 160, while the proximal end of the waveguide 152 is operably coupled to the TAG 200. The outer tube 154 is slidably disposed about the waveguide 152 and extends between the housing 110 and the end effector assembly 160. The rotating assembly 130 is rotatably mounted on the housing 110 and operably coupled to the elongated body portion 150 to enable rotation of the elongated body portion 150 and the end effector assembly 160 relative to the housing 110.

The end effector assembly 160 is disposed at a distal end of the elongated body portion 150 and includes the blade 162 and a jaw member 164. The jaw member 164 is pivotable relative to the blade 162 between an open position, in which, the jaw member 164 is spaced-apart from the blade 162, and a closed position, in which, the jaw member 164 is approximated relative to the blade 162 in juxtaposed alignment therewith for clamping tissue therebetween. The jaw member 164 is operably coupled to the distal end of the outer tube 154 and the proximal end of outer tube 154 is operably coupled to a movable handle 122 of the handle assembly 120, such that the jaw member 164 is movable between the open position and the closed position in response to actuation of the movable handle 122 of the handle assembly 120 relative to the fixed handle portion 124 thereof.

The blade 162 is configured to serve as an active or oscillating ultrasonic member that is selectively activatable to ultrasonically treat tissue grasped between the blade 162 and the jaw member 164. The TAG 200 is configured to convert electrical energy provided by the battery 300 into mechanical energy that is transmitted along the waveguide 152 to the blade 162. More specifically, the TAG 200 is configured to convert the electrical energy provided by the battery 300 into a high voltage alternating current (AC) waveform that drives the transducer (not shown) of the TAG 200. An activation button 140 is disposed on the housing 110 and is electrically coupled between the battery 300 and the TAG 200. The activation button 140 is selectively activatable in a first position and a second position to supply electrical energy from the battery 300 to the TAG 200 for operating instrument 100 in a low-power mode of operation and a high-power mode of operation, respectively.

With reference to FIGS. 1-3, inflow and return conduits 172, 174 extend from the cooling module 500 through the housing 110, and at least partially through the outer tube 154 of the elongated body portion 150. Proximal ends 173a, 175a of the respective inflow and return conduits 172, 174 are operably coupled to the cooling module 500.

With particular reference to FIGS. 2 and 3, the distal ends 173b, 175b of the respective inflow and return conduits 172, 174 extend into the waveguide 152. More specifically, the waveguide 152 defines a lumen 166 extending through a portion of the waveguide 152. The lumen 166 defines a closed distal end. The conduits 172, 174 enter the lumen 166 through an opening 168 defined within the waveguide 152 and disposed in communication with the lumen 166. A seal (not shown) is provided within the opening 168 and around the inflow and return conduits 172, 174 to inhibit escape of fluid therefrom. The inflow conduit 172 extends within the lumen 166. The return conduit 174 is disposed within the proximal end of the lumen 166. The inflow conduit 172 has a smaller diameter than the lumen 166 thereby defining an annular gap 169 therebetween to permit the return of fluid to the return conduit 174. As such, during cooling, a cooling fluid, such as, e.g., water or saline, is pumped through the inflow conduit 172 and is discharged through a distal end of the inflow conduit 172 at the distal end of the lumen 166. The cooling fluid then travels back proximally through the annular gap 169 and ultimately returns to the cooling module 500 (FIG. 1) via the return conduit 174. The inflow and return conduits 172, 174 may be at least partially formed of polyimide tubing. The portion of the inflow conduit 172 that extends distally through lumen 166 may be formed of stainless steel or other material suitable to withstand high temperatures.

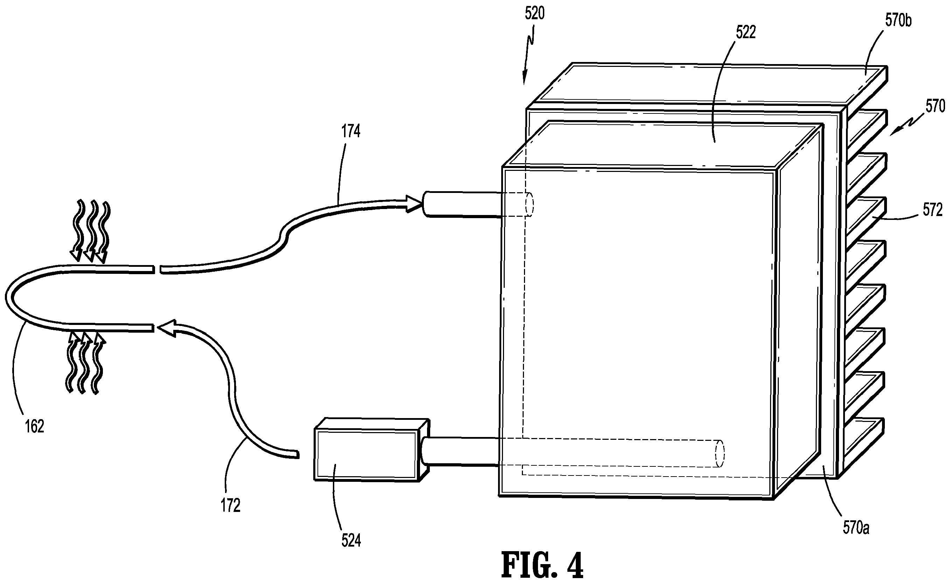

With reference to FIG. 3, the cooling module 500 (see also FIG. 4) includes an input port 510, the cooling assembly 520, a controller 530, and a user interface 540. The input port 510 enables operable coupling of the cable 400 with the cooling module 500. More specifically, the input port 510 includes an inflow conduit receptacle 512, a return conduit receptacle 514, and one or more electrical receptacles 516. The inflow conduit receptacle 512 operably couples the inflow conduit 172 with the cooling assembly 520 upon engagement of the cable 400 with the cooling module 500. The return conduit receptacle 514 operably couples the return conduit 174 with the cooling assembly 520 upon engagement of the cable 400 with the cooling module 500. The electrical receptacles 516 operably couple the TAG 200 with the controller 530 via the wires 410, upon engagement of the cable 400 with the cooling module 500. The inflow and return conduit receptacles 512, 514 each include one or more sensors 513, 515, respectively, associated therewith for sensing the temperature, the flow rate, and/or the presence of gas bubbles flowing therethrough.

With reference to FIG. 4, the cooling assembly 520 of the cooling module 500 includes a thermoelectric cooler 570, a fluid reservoir 522, and a pump 524. The inflow conduit 172 and the return conduit 174 are coupled to the cooling assembly 520. The thermoelectric cooler 570 is electrically coupled to the battery 300 (FIG. 1) or another power source to power the theromoelectrical cooler 570 and includes a cold side 570a and a hot side 570b. The cold side 570a is thermally coupled to the fluid reservoir 522 to transfer heat from the cooling fluid therein, and the hot side 570b includes a plurality of fins 572 to dissipate heat therefrom. The fluid reservoir 522 stores the cooling fluid to cool the blade 162 of the end effector assembly 160 (FIG. 1). The pump 524 supplies the cooling fluid from the fluid reservoir 522 through the blade 162 via the inflow and return conduits 172, 174 and the lumen 166 and may be configured to push the cooling fluid from the fluid reservoir 522 through the blade 162 and back to the fluid reservoir 522 or to pull the cooling fluid from the fluid reservoir 522 through the blade 162 and back to the fluid reservoir 522. Under either configuration, heat from the heated cooling fluid returning from the blade 162 to the fluid reservoir 522 via the return conduit 174 is transferred to the hot side 570b of the thermoelectric cooler 570 and dissipated through the plurality of fins 572. In this manner, a need for a large amount of fluid to cool the blade 162 is eliminated as the thermoelectric cooler 570 maintains the cooling fluid in the fluid reservoir 522 at a desired temperature for cooling the blade 162.

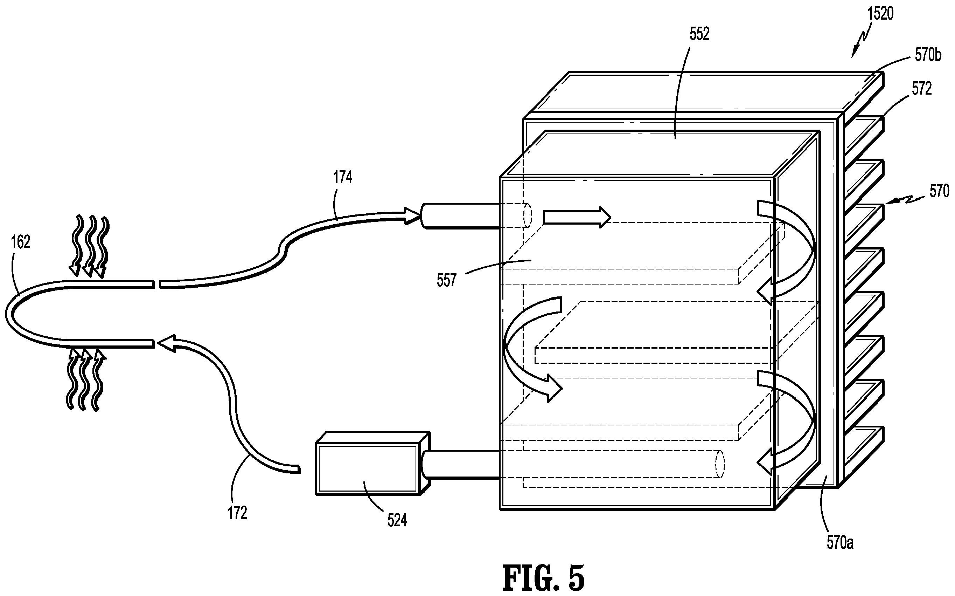

With reference now to FIG. 5, in another aspect of the present disclosure, the cooling assembly 1520 may include a fluid reservoir 552 having a plurality of dividers 557 defining a flow channel to guide the flow of the cooling fluid in the fluid reservoir 552. Dividers 557 may be arranged (as shown) to guide the flow of the cooling fluid horizontally along the cold side 570a of the thermoelectric cooler 570 in alternating directions from a top portion of the thermoelectric cooler 570 towards a bottom portion of the thermoelectric cooler 570. In this manner, the cooling fluid from the return conduit 174 enters the fluid reservoir 552 at an upstream of the fluid reservoir 552, and the cooling fluid is supplied to the blade 162 from a downstream of the fluid reservoir 552. Under such a configuration, the upstream of the fluid reservoir 552 is spaced part from the downstream of the fluid reservoir 552. In this manner, the cooling fluid in the fluid reservoir 552 may be more efficiently cooled and supplied to the blade 162. Other suitable configurations for guiding the flow of the cooling fluid are also contemplated such as, e.g., guiding the cooling fluid vertically along the cold side 570a of the thermoelectric cooler 570 in alternating directions from one side portion of the thermoelectric cooler 570 towards another side portion of the thermoelectric cooler 570.

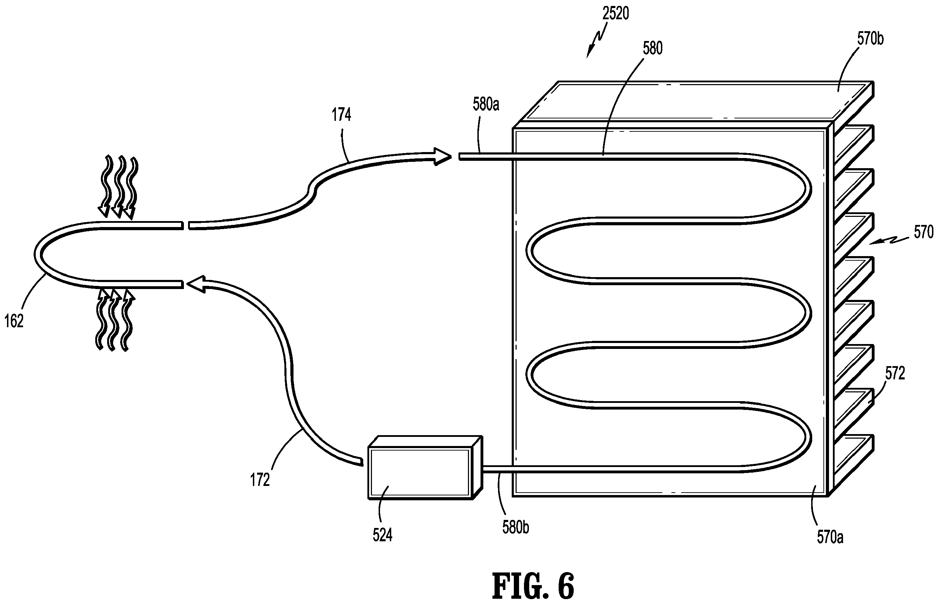

With reference to FIG. 6, in yet another aspect of the present disclosure, a cooling assembly 2520 may be used with the cooling module 500. In particular, the cooling assembly 2520 includes the pump 524, the thermoelectric cooler 570, and a fluid line 580 thermally coupled to the cold side 570a of the thermoelectric cooler 570. Under such a configuration, the cooling assembly 2520 eliminates a fluid reservoir (although a fluid reservoir may be provided at the inflow and/or outflow connections between fluid line 580 and conduits 172, 174) and utilizes the fluid line 580 that interconnects and is in fluid communication with the inflow and return conduits 172, 174. A first end 580a of the fluid line 580 is coupled to the return conduit 174 to receive the cooling fluid used to cool the blade 162. The fluid line 580 is thermally coupled to the cold side 570a of the thermoelectric cooler 570. For example, the fluid line 580 may be supported on the cold side 570a in a coiled manner or other suitable manner to increase the length of fluid line 580 extending along the cold side 570a of the thermoelectric cooler 570. Under such a configuration, heat from the cooling fluid returning to the fluid line 580 via the return conduit 174 is transferred to the hot side 570b of the thermoelectric cooler 570 and is dissipated through the plurality of fins 572. The cooling fluid exits through a second end 580b of the fluid line 580 and is supplied to the blade 162 via the inflow conduit 172. The pump 524 circulates the cooling fluid from the fluid line 580 through the blade 162 via the inflow conduit 172 and back to the fluid line 580 via the return conduit 174 and the lumen 166. Under such a configuration, a need for a large amount of fluid to cool the blade 162 is eliminated as thermoelectric cooler 570 directly cools the fluid in the fluid line 580 at a desired temperature for cooling the blade 162.

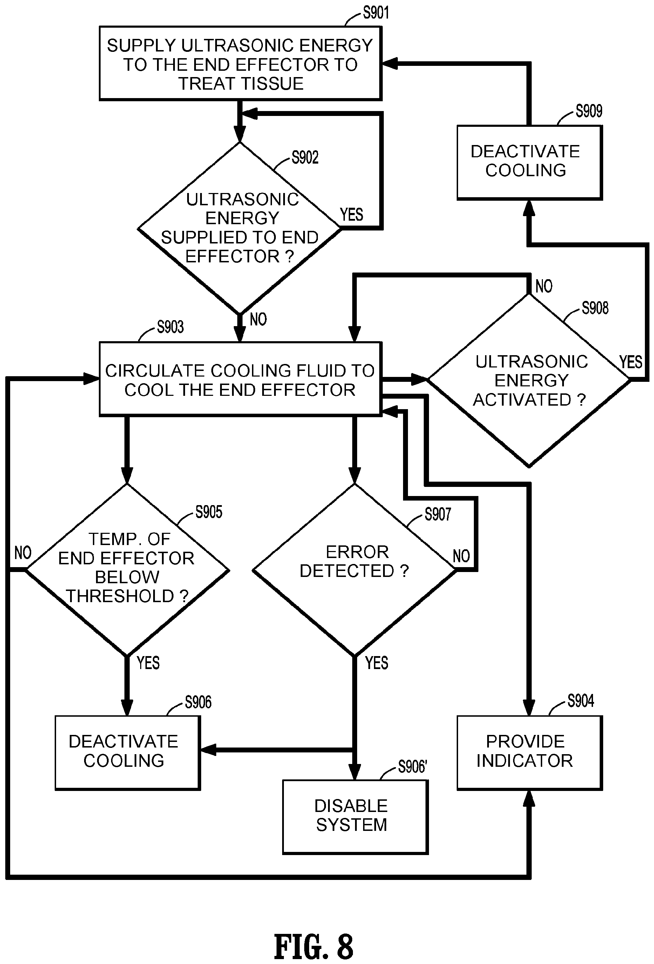

With reference back to FIG. 3, the controller 530 of the cooling module 500 includes a processor 532 and a memory 534 storing instructions for execution by the processor 532. The controller 530 may be operatively coupled to the cooling assembly 520, 1520, or 2520, the sensors 513, 515, the TAG 200 (via wires 410 extending through cable 400), and the user interface 540. The controller 530 may be configured to implement the method of FIG. 8 to instruct the cooling assembly 520, 1520, or 2520 to activate the pump 524, to thereby initiate or stop blade cooling, based at least on feedback received from the sensors 513, 515, the TAG 200, or other received feedback. As detailed below, the controller 530 may be configured to instruct the cooling assembly 520, 1520, 2520 to turn OFF the pump 524 when the sensor 515 indicates a sufficiently low temperature of the cooling fluid returning from the return conduit 174. The temperature of the blade 162 (FIG. 3) of the end effector assembly 160 may be extrapolated from the temperature of the cooling fluid returning from the return conduit 174, or the temperature difference between the cooling fluid entering inflow conduit 172 and the cooling fluid exiting the return conduit 174. Accordingly, the pump 524 may be turned OFF upon the blade 162 reaching a sufficiently cool temperature, e.g., below about 60 °C (or other suitable temperature threshold), as indicated by a sufficiently low return fluid temperature or sufficiently small temperature differential. As an alternative to the sensors 513, 515 sensing temperature at the input port 510, temperature sensors may be incorporated into the cooling assembly 520, 1520, or 2520 for similar purposes as noted above. Further, a thermocouple 167 (FIG. 4) or other suitable temperature sensor may additionally or alternatively be incorporated into the blade 162 to detect temperature at the blade 162.

The controller 530 may additionally or alternatively instruct the cooling assembly 520, 1520, or 2520 to turn OFF the pump 524 or disable the entire system when the sensor 513 and/or sensor 515 indicates an error. Such errors may include, for example, where the sensor 513 and/or the sensor 515 detects a flow rate through the inflow conduit 172 and/or return conduit 174 below a threshold flow rate, and/or when the sensor 515 detects gas bubbles, or a gas bubble volume greater than a threshold volume, returning from the return conduit 174. Reduced flow rate and/or the presence of gas bubbles (or a greater volume of gas bubbles) may be an indication of a blockage or leak within the fluid flow path or damage to one of the inflow or return conduits 172, 174 and, thus, the circulation of fluid is stopped by turning OFF the pump 524 when such condition is detected.

The controller 530 may further be configured, as also detailed below with respect to FIG. 8, to output an appropriate signal to the user interface 540 and/or the indicator 202 of the TAG 200 to alert the user that blade cooling is in effect, e.g., that that pump 524 is ON, that an error, e.g., a blockage or leakage, has occurred, and/or that the blade 162 (FIG. 2) has been sufficiently cooled and is ready for further use. The user interface 540 and/or indicator 202 may provide such an alert in the form of audible, visual, and/or tactile output.

The controller 530 may be configured to communicate with the TAG 200 to determine whether ultrasonic surgical instrument 100 is in use, e.g., whether activation button 140 is actuated such that ultrasonic energy is being supplied to the blade 162 (see FIG. 1), and to control the cooling assembly 520, 1520, or 2520. More specifically, the controller 530 may instruct the cooling assembly 520, 1520, or 2520 to turn OFF the pump 524 when the ultrasonic surgical instrument 100 is in use. Once the use is complete, the pump 524 may be turned ON for a pre-determined time, until the blade 162 has been sufficiently cooled, an error is detected, or the ultrasonic surgical instrument 100 is once again put into use.

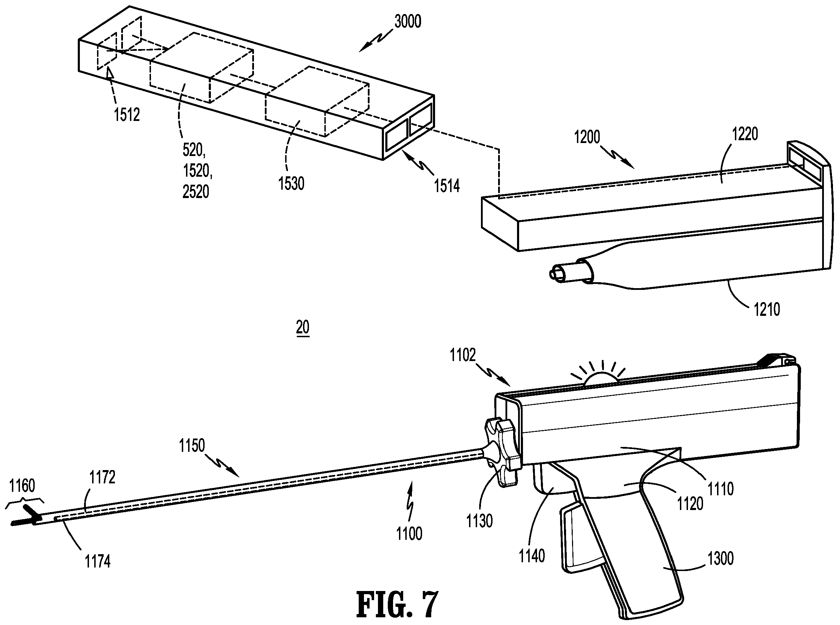

Turning now to FIG. 7, a surgical system 20 is similar to the surgical system 10 (FIG. 1) and generally includes an ultrasonic surgical instrument 1100 and a cooling module 3000. In particular, the cooling module 3000 is formed as part of the disposable 1102 or releasably mounted on the ultrasonic surgical instrument 1100.

The ultrasonic surgical instrument 1100 generally includes a disposable 1102, a transducer and generator assembly ("TAG") 1200 including a transducer 1210 and a generator 1220, and a battery 1300. The disposable 1102 includes a housing 1110, a handle assembly 1120, a rotating assembly 1130, an activation button 1140, an elongated body portion 1150, and an end effector assembly 1160, each of which are similar to the corresponding components of the ultrasonic surgical instrument 100 (FIG. 1). The TAG 1200 and the battery 1300 are releasably engageable with the housing 1110 of the disposable 1102. The TAG 1200 and the battery 1300 are similar to those detailed above with respect to the ultrasonic surgical instrument 100 (FIG. 1).

The cooling module 3000 is similar to the cooling module 500 (FIG. 1) and includes input ports 1512, 1514, the cooling assembly 520, 1520, or 2520, and the controller 1530. The cooling module 3000 may be permanently mounted on the TAG 1200. Alternatively, the cooling module 3000 may be releasably engageable with both the TAG 1200 and the disposable 1102. The input port 1512 enables operable coupling of the cooling assembly 520, 1520, or 2520 with the conduits 1172, 1174 of the ultrasonic surgical instrument 1100, while the input port 1514 enables communication between the controller 1530 and the generator 1220, both of which are similar as detailed above with respect to the input port 510 (FIG. 4). The controller 1530 is also similar to the controller 530 described hereinabove, and may be configured to operate in a similar manner as mentioned above.

Turning now to FIG. 8, a method in accordance with the present disclosure is detailed for use with any suitable surgical instrument, e.g., ultrasonic surgical instruments 100 (FIG. 1) or 1100 (FIG. 7). Initially, at S901, the instrument is activated to supply ultrasonic energy to the end effector to treat, for example, coagulate and/or cut, tissue. At S902 it is determined whether ultrasonic energy is still being supplied to the end effector. Such a determination may be performed by determining whether an activation button is activated. However, other suitable ways of determining whether ultrasonic energy is being supplied to the end effector are also contemplated, e.g., monitoring the output of the battery (or other power source) or the input to or output from the transducer. If it is determined that ultrasonic energy is being supplied, the determination at S902 is repeatedly made, periodically or continuously, until it is determined that ultrasonic energy is no longer being supplied to the end effector.

Once it is determined that ultrasonic energy is no longer being supplied to the end effector, the cooling system including a thermoelectric cooler that is thermally coupled to a fluid reservoir or a fluid line is activated as indicated in S903, to circulate cooling fluid through the end effector to cool the end effector. Likewise, an indicator S904 is provided to indicate that cooling is ongoing. During cooling, it is determined, at S905, whether the temperature of the end effector is below a threshold temperature. The temperature of the end effector may be determined indirectly by detecting the temperature of the cooling fluid output to the end effector and returning therefrom.

If the temperature of the end effector is determined to be above the threshold temperature, cooling continues at S903 and the temperature is continuously or periodically determined at S905. At the same time, an indicator, as indicated in S904, is provided to alert the user that cooling is still ongoing. Once the temperature of the end effector is below the threshold temperature, as indicated in S906, cooling is deactivated and the indicator is removed.

Referring to S907, during cooling, if an error is detected, cooling is deactivated at S906 and an indicator is provided at S904. Alternatively, the entire system may be shut down, inhibiting further activation or use, as indicated at S906. An error may include, as noted above, a condition where the flow rate of the cooling fluid is below a flow rate threshold, a condition where the cooling fluid includes gas bubbles or a sufficiently high volume of gas bubble, or other suitable error condition. The indicator provided in response to an error may be different from the indicator provided during cooling. If no error is detected, cooling continues at S903.

Turning to S908, during cooling, it is determined whether the supply of ultrasonic energy to the end effector has been activated. If so, cooling is deactivated at S909 and the method returns to S901. If the supplying of ultrasonic energy to the end effector has not been activated, the method returns to S903 and cooling is continued until the temperature of the end effector is below the threshold temperature, an error is detected, or the supply of ultrasonic energy to the end effector is activated.

While several embodiments of the disclosure have been shown in the drawings and described hereinabove, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical system comprising:
   a surgical instrument including a housing and an elongated portion extending from the housing and supporting an end effector; and
   a cooling module operably coupled to the surgical instrument, the cooling module including:
      a fluid reservoir retaining a cooling fluid;
      a thermoelectric cooler having a first portion thermally coupled to the fluid reservoir and a second portion including a plurality of fins dissipating heat therefrom; and
      a pump assembly supplying the cooling fluid through at least a portion of the surgical instrument and back to the fluid reservoir, thereby defining a flow path to cool the end effector of the surgical instrument.
2. The surgical system according to paragraph 1, wherein the cooling module is external of the surgical instrument.
3. The surgical system according to paragraph 1, wherein the fluid reservoir includes dividers to guide flow of the cooling fluid.
4. The surgical system according to paragraph 1, wherein the cooling module is disposed within the housing of the surgical instrument.
5. The surgical system according to paragraph 1, wherein the surgical instrument includes an ultrasonic waveguide having a blade at a distal end thereof.
6. The surgical system according to paragraph 5, wherein the flow path extends at least partially through the blade.
7. The surgical system according to paragraph 5, wherein the surgical instrument further includes an ultrasonic transducer coupled to the ultrasonic waveguide to energize the blade for treating tissue.
8. The surgical system according to paragraph 1, further comprising a generator configured to supply energy to the surgical instrument.
9. The surgical system according to paragraph 8, wherein the generator is disposed on the surgical instrument.
10. The surgical system according to paragraph 8, wherein the generator is spaced-apart from the surgical instrument.
11. The surgical system according to paragraph 1, further comprising a controller configured to determine a temperature of the surgical instrument.
12. The surgical system according to paragraph 11, wherein the controller is configured to control the pump assembly.
13. A surgical system comprising:
   a surgical instrument including a housing, an end effector operably coupled to the housing, and first and second conduits thermally coupled to the end effector; and
   a cooling module disposed within the housing, the cooling module including:
      a fluid line retaining a cooling fluid therein and interconnecting the first and second conduits, the fluid line being in fluid communication with the first and second conduits thereby defining a closed fluid path;
      a cooling fluid disposed in the closed fluid path;
      a thermoelectric cooler having a first portion thermally coupled to the fluid line and a second portion including a plurality of fins to dissipate heat therefrom; and
      a pump assembly circulating the cooling fluid through the closed fluid path to cool the end effector of the surgical instrument.
14. The surgical system according to paragraph 13, wherein the surgical instrument includes an ultrasonic waveguide having a blade defined at a distal end thereof.
15. The surgical system according to paragraph 14, wherein the closed fluid path extends at least partially through the blade.
16. The surgical system according to paragraph 14, wherein the surgical instrument further includes an ultrasonic transducer coupled to the ultrasonic waveguide and configured to energize the blade for treating tissue therewith.
17. The surgical system according to paragraph 14, further comprising a generator configured to supply energy to the surgical instrument.
18. The surgical system according to paragraph 13, further comprising a controller configured to control the pump assembly.

## Claims

1. A surgical system comprising:
a surgical instrument including a housing and an elongated portion extending from the housing and supporting an end effector; and
a cooling module operably coupled to the surgical instrument, the cooling module including:
a fluid reservoir retaining a cooling fluid;
a thermoelectric cooler having a first portion thermally coupled to the fluid reservoir and a second portion including a plurality of fins dissipating heat therefrom; and
a pump assembly supplying the cooling fluid through at least a portion of the surgical instrument and back to the fluid reservoir, thereby defining a flow path to cool the end effector of the surgical instrument.

2. The surgical system according to claim 1, wherein the cooling module is external of the surgical instrument.

3. The surgical system according to claim 1 or 2, wherein the fluid reservoir includes dividers to guide flow of the cooling fluid.

4. The surgical system according to any preceding claim, wherein the cooling module is disposed within the housing of the surgical instrument.

5. The surgical system according to any preceding claim, wherein the surgical instrument includes an ultrasonic waveguide having a blade at a distal end thereof, preferably wherein the flow path extends at least partially through the blade yet further preferably wherein the surgical instrument further includes an ultrasonic transducer coupled to the ultrasonic waveguide to energize the blade for treating tissue.

6. The surgical system according to any preceding claim, further comprising a generator configured to supply energy to the surgical instrument preferably wherein the generator is disposed on the surgical instrument.

7. The surgical system according to claim 6, wherein the generator is spaced-apart from the surgical instrument.

8. The surgical system according to any preceding claim, further comprising a controller configured to determine a temperature of the surgical instrument.

9. The surgical system according to claim 8, wherein the controller is configured to control the pump assembly.

10. A surgical system comprising:
a surgical instrument including a housing, an end effector operably coupled to the housing, and first and second conduits thermally coupled to the end effector; and
a cooling module disposed within the housing, the cooling module including:
a fluid line retaining a cooling fluid therein and interconnecting the first and second conduits, the fluid line being in fluid communication with the first and second conduits thereby defining a closed fluid path;
a cooling fluid disposed in the closed fluid path;
a thermoelectric cooler having a first portion thermally coupled to the fluid line and a second portion including a plurality of fins to dissipate heat therefrom; and
a pump assembly circulating the cooling fluid through the closed fluid path to cool the end effector of the surgical instrument.

11. The surgical system according to claim 10, wherein the surgical instrument includes an ultrasonic waveguide having a blade defined at a distal end thereof.

12. The surgical system according to claim 11, wherein the closed fluid path extends at least partially through the blade.

13. The surgical system according to claim 12, wherein the surgical instrument further includes an ultrasonic transducer coupled to the ultrasonic waveguide and configured to energize the blade for treating tissue therewith.

14. The surgical system according to any of claims 10 to 13, further comprising a generator configured to supply energy to the surgical instrument.

15. The surgical system according to any one of claims 10 to 14, further comprising a controller configured to control the pump assembly.
